(19) Europäisches Patentamt
European Patent Office
Office européen des brevets

(11) Publication number : **0 345 931 B1**

(12) **EUROPEAN PATENT SPECIFICATION**

(45) Date of publication of patent specification : **20.01.93 Bulletin 93/03**

(21) Application number : **89304068.3**

(22) Date of filing : **24.04.89**

(51) Int. Cl.$^5$ : **A61K 45/06,** A61K 31/55, A61K 31/38, A61K 31/34, // (A61K31/55, 31:44, 31:38, 31:34), (A61K31/55, 31:445, 31:40, 31:38, 31:34)

(54) **Certain PAF antagonist/antihistamine combinations and methods.**

Consolidated with 89905952.1/0402422 (European application No./publication No.) by decision dated 18.03.91.

(30) Priority : **27.04.88 US 186535**

(43) Date of publication of application : **13.12.89 Bulletin 89/50**

(45) Publication of the grant of the patent : **20.01.93 Bulletin 93/03**

(84) Designated Contracting States : **AT BE CH DE ES FR GB GR IT LI LU NL SE**

(56) References cited :
**EP-A- 0 154 887**
**DE-A- 3 502 392**
**US-A- 4 539 332**

(73) Proprietor : **SCHERING CORPORATION**
**2000 Galloping Hill Road**
**Kenilworth New Jersey 07033 (US)**

(72) Inventor : **Billah, Motasim M.**
**4 Patriot Court**
**Edison New Jersey 08820 (US)**

(74) Representative : **Ritter, Stephen David et al**
**Mathys & Squire 10 Fleet Street**
**London EC4Y 1AY (GB)**

EP 0 345 931 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

BACKGROUND OF THE INVENTION

The present invention relates to synergistic combinations of certain diaryl tetrahydrofuran, diaryl tetrahydrothiophene, triazolobenzodiazepine or thienotriazolodiazepine derivatives with antihistamines and the use thereof to treat allergic reactions.

Several mediators possessing a broad spectrum of potent biological activities are released during allergic reactions. Prominent among these mediators are histamine, leukotrienes and platelet-activating factor (PAF).

Compounds which prevent the effects of the mediators are thus of interest in treating allergic reactions. For example, numerous antihistamines are known in the art. Chlorpheniramine, brompheniramine, clemastine, ketotifen, azatadine, loratadine, and terfenadine are examples of commercially available or soon to be available antihistamines.

Various compounds have been disclosed as PAF antagonists. For example, compounds of the formula Ia or Ib

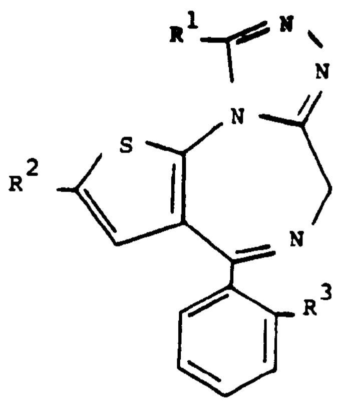

Ia

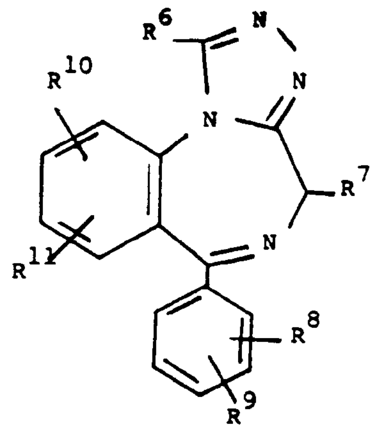

Ib

(wherein $R^1$, $R^2$, $R^3$, $R^6$, $R^7$, $R^8$, $R^9$, $R^{10}$ and $R^{11}$ are as defined below) are disclosed as PAF antagonists in Science, Vol. 226, p. 1454 (1984) and German Offenlegungsschrift DE-A- 35 02 392 A1.

Compounds of the formula II

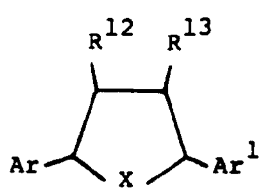

II

(wherein $R^{12}$ $R^{13}$, Ar, $Ar^1$ and X are as defined below) are also disclosed as PAF antagonists in Biftu et al., U.S. Patent Nos. US-A 4,539,332 and US-A- 4,595,693 and European Patent Application No. EP-A- 0 154 887

Antihistamines have proven useful in the treatment of certain allergic disorders such as seasonal rhinitis. However, the antihistamine therapy is ineffective in such complex allergic disorders as asthma indicating that histamine is only one of several mediators released during an allergic response. Indeed, in guinea pigs, allergic bronchospasm appears to be composed of three distinct components, mediated separately by histamine, leukotrienes and PAF.

PAF shares with histamine the abilities to cause bronchospasm and vasopermeability. In addition, PAF induces non-specific bronchial hyperreactivity in man as well as in animals.

Touvay et al., 6th International Conference on Prostaglandins, Florence, Italy, June 3-6, 1986, p. 914, disclose BN1267 and related compounds of the general formula

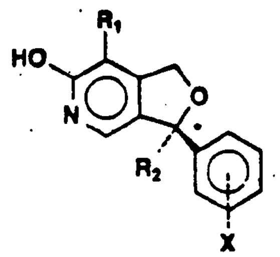

which compounds are said to exhibit antihistaminic activity. The presentation also mentions that "...the association of this dose [10 mg/kg p.o.] of BN 1267 with a non-active dose of a specific PAF-receptor antagonist BN 52021 (-10% at 2 mg/kg p.o.) administered one hour before PAF-acether significantly antagonized the bronchospasm (-45% $p < 0.01$)." BN 52021 is ginkgolide B having the structure:

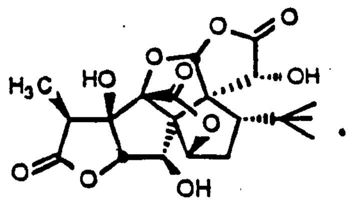

While we have found that representative examples of the above PAF antagonists prevent PAF-induced lethality, we have also found that they do not provide protection against allergic death (anaphylactic shock) in sensitized mammals, such as ovalbumin sensitized mice. Antihistamines such as chlorpheniramine, clemastine and ketotifen provided only partial protection against ovalbumin-induced lethality.

## SUMMARY OF THE INVENTION

It has now surprisingly been found that complete or at least potentiated protection against allergic reactions such as antigen-induced death in mammals can be achieved by administering to such mammal an anti-allergic effective amount of a combination comprising

(1) a PAF-antagonist

(a) of the formula Ia or Ib

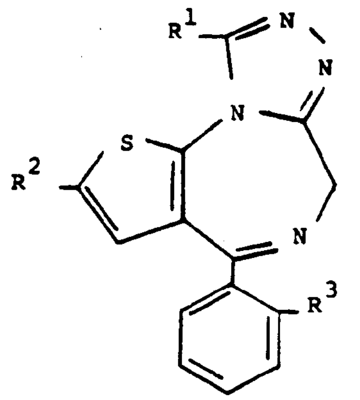

Ia

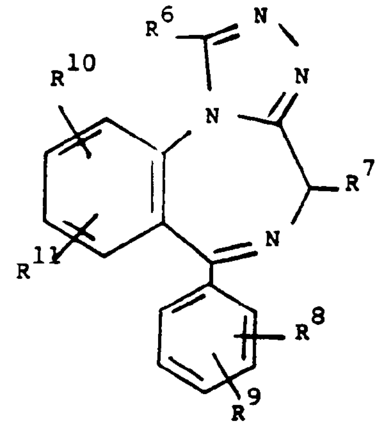

Ib

or a pharmaceutically acceptable salt or solvate of a compound Ia or Ib, wherein:

$R^1$ is H, lower alkyl of 1 to 4 carbon atoms, cyclopropyl, methoxy, chloro or bromo,

$R^2$ represents

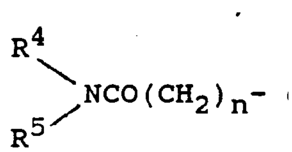

or halo,

$R^3$ represents H, chloro or bromo,

$R^4$ and $R^5$ may be the same or different and each is independently selected from H, alkyl of 1 to 4 carbon atoms or hydroxyalkyl having from 1 to 4 carbon atoms, or $R^4$ and $R^5$ together with the nitrogen atom attached thereto represent a 5, 6 or 7 membered, saturated ring, which ring optionally contains another heteroatom or hetero group selected from -O-, -S-, -NH- or $-N(CH_3)-$,

n represents 0, 1, 2, 3, 4, 5, 6, 7 or 8,

$R^6$ represents H, lower alkyl of 1 to 3 carbon atoms, phenyl, benzyl or COOR′,

$R^7$ represents H or alkyl of 1 to 3 carbon atoms,

$R^8$, $R^9$, $R^{10}$ and $R^{11}$ may be the same or different and each independently represents H, alkyl of 1 to 3 carbon atoms, halo, nitro, cyano, trifluoromethyl, alkoxy, alkylthio, alkylsulfinyl, alkylsulfonyl, alkanoylamino or dialkylamino, wherein each alkyl portion of alkoxy, alkylthio, alkylsulfinyl, alkylsulfonyl, alkanoylamine or dialkylamino contains 1 to 3 carbon atoms,

R′ represents alkyl of 1 to 4 carbon atoms; or

(b) of formula II

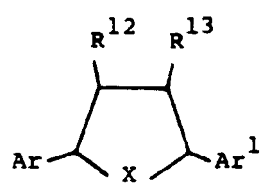

II

wherein:

X is O, S, SO, or $SO_2$,

$R^{12}$ and $R^{13}$ may be the same or different and each independently represents H, lower alkyl of 1 to 6 carbon atoms, halo, halo-lower alkyl of 1 to 6 carbon atoms, $CONR^{14}R^{15}$, lower alkenyl of 1 to 6 carbon atoms, lower alkynyl of 1 to 6 carbon atoms, $COR^{14}$, $COOR^{14}$, $CH_2OR^{14}$, $CH_2NR^{14}R^{15}$, $CH_2SR^{14}$,

=O or $OR^{15}$,

Ar and $Ar^1$ may be the same or different and each independently represents pyrryl, furyl, pyridyl, thienyl, cyclohexyl, naphthyl or a group of the formula

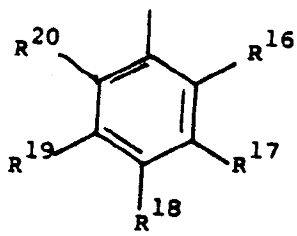

$R^{14}$ and $R^{15}$ may be the same or different and each is independently H or lower alkyl of 1 to 6 carbon atoms;

$R^{16}$, $R^{17}$, $R^{18}$, $R^{19}$ and $R^{20}$ may be the same or different and each is independently H, $-OR^{14}$, $-SR^{14}$, $-SOR^{14}$, $-SO_2R^{14}$, $-OCF_3$, $-SCF_3$, $-NR^{14}R^{15}$, $-OCH_2CO_2R^{14}$, $-SO_2NR^{14}R^{15}$, $-CO_2R^{14}$, $-N(R^{14})SO_2R^{15}$, $COR^{14}$, $NO_2$ or CN, or $R^{16}$ and $R^{17}$, $R^{17}$ and $R^{18}$, $R^{18}$ and $R^{19}$ or $R^{19}$ and $R^{20}$ together represent a $-OCH_2O-$, $-OCH_2CH_2O-$ or $-OCH_2CH_2NR^{14}-$ bridge; and

(2) an antihistamine, which is preferably selected from chlorpheniramine, brompheniramine, clemastine, ketotifen, azatadine, loratadine, terfenadine, cetirizine, astemizole, tazifylline, levocabastine, diphenhydramine, temelastine, etolotifen, acrivastine, azelastine, ebastine, mequitazine, and the compounds

3-amino-9,13b-dihydro-1H-dibenz[c,f]imidazo [1,5-a]azepine[WAL-801CL],

1-(2-ethoxyethyl)-2-(4-methyl-1-homopiperazinyl)benzimidazole[KB-2413],

2-(dimethylamino)ethyl-2,3-dihydro-4-methylpyrido-[3,2-f]-1,4-oxazepine-5(4H)-thione[AHR 11325], or a pharmaceutical acceptable salt of such compounds.

The present invention thus provides the synergistic use of PAF antagonist of formula Ia, Ib or II (above) in combination with an antihistamine for the manufacture of a medicament for treating allergy.

The present invention also contemplates a pharmaceutical composition comprising such synergistic combination together with a pharmaceutically acceptable carrier and a method for making said composition.

In the compounds of formula Ia, $R^1$ is preferably $CH_3$ and $R^3$ is preferably chloro or bromo. $R^2$ is preferably Br or $R^4R^5NCO(CH_2)_n$. $R^4$ and $R^5$ are preferably selected from H, methyl, ethyl or hydroxyethyl or $R^4$ and $R^5$ together represent morpholino, and n is preferably 2.

In one embodiment of the use or composition of the invention, the compound of formula Ia or Ib is selected from

8-chloro-1-methyl-6-phenyl-4H-s-triazolo[4,3-a][1,4]benzodiazepine,

8-chloro-1-methyl-6-(o-fluorophenyl)-4H-s-triazolo[4,3-a][1,4]benzodiazepine,

1-methyl-6-phenyl-4H-s-triazolo[4,3-a][1,4]benzodiazepine,

8-chloro-1-propyl-6-phenyl-4H-s-triazolo[4,3-a][1,4]benzodiazepine,

8-chloro-1-isopropyl-6-phenyl-4H-s-triazolo[4,3-a][1,4]benzodiazepine,

8-chloro-1-ethyl-6-phenyl-4H-s-triazolo[4,3-a][1,4]benzodiazepine,

1-methyl-6-phenyl-8-(trifluoromethyl)-4H-s-triazolo[4,3-a][1,4]benzodiazepine,

8-chloro-1-methyl-6-(o-chlorophenyl)-4H-s-triazolo[4,3-a][1,4]benzodiazepine,

4-(2-chlorophenyl)-9-methyl-6H-thieno[3,2-f][1,2,4]triazolo[4,3-a][1,4]diazepine-2-propionic acid morpholide,

4-(2-chlorophenyl)-9-methyl-6H-thieno[3,2-f][1,2,4]triazolo[4,3-a][1,4]diazepine-2-propionamide,

4-(2-chlorophenyl)-9-cyclopropyl-6H-thieno[3,2-f][1,2,4]triazolo[4,3-a][1,4]diazepine-2-propionic acid morpholide, or

4-(2-chlorophenyl)-9-methyl-6H-thieno[3,2-f][1,2,4]triazolo[4,3-a][1,4[diazepine-2-propionic acid diethyl amide.

Preferably, the compound of formula Ia or Ib is

4-(2-chlorophenyl)-9-methyl-6H-thieno[3,2-f][1,2,4]triazolo[4,3-a][1,4]diazepine-2-propionic acid morpholide (WEB 2086),

8-chloro-6-(2-chlorophenyl)9-1-methyl-4H-s-triazolo[4,3-a][1,4]benzodiazepine (triazolam), or

8-chloro-1-methyl-6-phenyl-4H-3-triazolo-[4,3-a][1,2]benzodiazepine (alprazolam).

In another embodiment of the composition of the invention, the PAF antagonist is of the formula IIa, IIb or IIc:

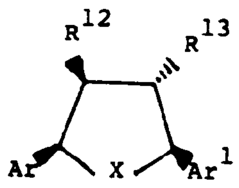

IIa

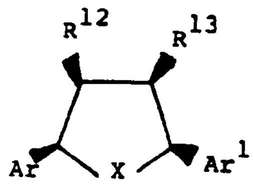

IIb

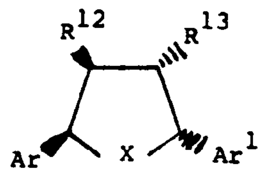

IIc

wherein X, $R^{12}$, $R^{13}$, Ar and $Ar^1$ are as defined above. Preferably, Ar and $Ar^1$ independently represent 2,4-dimethoxyphenyl or 3,4,5-dimethoxyphenyl. X is preferably O or S, more preferably S. $R^{12}$ and $R^{13}$ preferably independently represent H or $CH_3$.

Suitable compounds of formula II for use in the present method and composition include

2,5-bis(3,4-dimethoxyphenyl)tetrahydrofuran,

2,5-bis(3,4,5-trimethoxyphenyl)tetrahydrofuran,

2,5-bis(3,4,5-trimethoxyphenyl)tetrahydrothiophene, and

2-(3-methoxy-5-methylsulfonyl-4-propoxyphenyl)-5-(3,4,5-trimethoxyphenyl)tetrahydrofuran, preferably in their respective trans forms. 3,4-Dimethylated forms of such compounds are also contemplated. One preferred compound is trans-2,5-bis(3,4,5-trimethoxyphenyl)-tetrahydrofuran (L-652,731).

The antihistamine employed in the use and composition of the invention is preferably selected from chlorpheniramine, ketotifen, loratadine, terfenadine, clemastine or astemizole.

## DETAILED DESCRIPTION OF THE INVENTION

The compounds of formula Ia and Ib are known, e.g. from German Offenlegungsschrift No. 35 02 392 A1 and U.S. patent Nos. 3,987,052, and 3,980,790 as are the methods for preparing such compounds.

The compounds of formula II and the methods for preparing such compounds are also known, e.g., from U.S. Patent Nos. 4,595,693 and 4,539,322 and in European Patent Application No. 0 154 887 A1.

Any antihistamine can be employed in the present invention. Suitable antihistamines include chlorpheniramine, bromopheniramine, clemastine, ketotifen, azatadine, loratadine, terfenadine, cetirizine, astemizole, tazifylline, levocabastine, diphenhydramine, temelastine, etolatifen, acrivastine, azelastine, ebastine, mequitazine, and the compounds

3-amino-9,13b-dihydro-1H-dibenz[c,f] imidazo[1,5-a]azepine[WAL-801CL],

1-(2-ethoxyethyl)-2-(4-methyl-1-homopiperazinyl)benzimidazole[KB-2413],

2-(dimethylamino)ethyl-2,3-dihydro-4-methylpyrido-[3,2-f]-1,4-oxazepine-5(4H-thione[AHR 11325], or a pharmaceutical acceptable salt of such compounds.

The compositions and use of the invention can be used to treat allergic disorders such as seasonal rhinitis, perennial vasomotor rhinitis, acute utricaria, chronic utricaria, atopic dermatitis, contact dermatitis, itching pruritides, angioedema, conjunctivitis, chronic bronchitis, systemic anaphylaxis, serum sickness, and bronchial asthma.

One of the characteristic features of asthma that also occurs in some patients with chronic bronchitis and allergic rhinitis is extreme sensitivity of the airways (airway hyperreactivity) to physical, chemical and pharmacological agents. These patients develop a greater degree of bronchospasm to a wider variety of stimuli than do healthy subjects. PAF causes not only acute bronchospasm but also non-specific, sustained airway hyperreactivity in man. Lung inflammation is believed to be central to the establishment of airway hyperreactivity. PAF is a potent mediator of inflammation and histamine, by virtue of its ability to cause vasopermeability, may exacerbate the inflammatory process initiated by PAF. Thus, PAF, either alone or in combination with histamine, might play a critical role in the pathogenesis of asthma and related disorders. Apparently, from our findings inhibition of PAF could not only enhance the efficacy of antihistamine therapy, but also offer additional, new utilities for antihistamines. For instance, the combined use of anti-PAF and antihistaminic agents may be useful in correcting the underlying cause of asthma.

The anti-allergic effect of the combinations of PAF antagonists and antihistamines of the present invention may be demonstrated by the test procedures described below:

PAF-INDUCED LETHALITY IN MICE AND EVALUATION OF IN VIVO ANTI-PAF ACTIVITY OF VARIOUS PHARMACOLOGICAL AGENTS:

Swiss-Webster female CFW mice (from Charles River), 5-10 per group, were injected through the tail vein with PAF in 0.5 ml of saline containing bovine serum albumin, and death was recorded. PAF at doses of 100 µg/kg, 150 µg/kg and 200 µg/kg causes 0, 60% and 100% lethality, respectively, and death occurred within 30 minutes. L-652,731 (A), WEB 2086 (B), chlorpheniramine (C) and ketotifen (D) suspended 0.4% methylcellulose vehicle were given intraperitoneally (0.5 ml) 30 minutes before PAF injection. Death occurring within 1 hour after PAF challenge was recorded and the results are shown in Table I below as % lethality.

TABLE I

EFFECTS OF VARIOUS ANTAGONISTS ON PAF-INDUCED LETHALITY IN MICE

| Compound[a] | Dose (mg/kg) | Number of animals | % Lethality | $ED_{50}$ (mg/kg) |
|---|---|---|---|---|
| none | - | 38 | 95 | - |
| A | 5 | 6 | 84 | |
| | 10 | 18 | 72 | 20 |
| | 25 | 12 | 25 | |
| | 50 | 18 | 16 | |
| B | 1 | 6 | 84 | |
| | 2 | 5 | 20 | 1.5 |
| | 5 | 6 | 16 | |
| | 10 | 6 | 0 | |
| C | 10 | 6 | 100 | - |
| D | 1.4 | 8 | 100 | - |

[a]Compounds given i.p. 30 minutes before i.v. challenge with PAF (200 µg/kg). Death was recorded 1 hour after the challenge.

SENSITIZATION OF MICE AND INDUCTION OF ANAPHYLACTIC DEATH:

Swiss-Weber female CFW mice (20-30 g) from Charles River were acclimatized for 7-14 days. Animals were sensitized by injecting intraperitoneally 0.5 ml of saline containing 1.6 mg of ovalbumin adsorbed to 100 mg of $Al(OH)_3$ gel. Ten to 15 days after sensitization, the animals, at least six per group, were injected intraveneously through the tail vein with 1.6 mg of ovalbumin in 0.5 ml of saline. One hundred percent of the sensitized animals died within 10-20 minutes. Injection of ovalbumin into normal mice caused no lethality. Compounds L-652,731 (A), WEB 2086 (B), chlorpheniramine (C), ketotifen (D) and clemastine (E), and conbinations A+C, B+C, A+D, B+D, A+E amd B+E were suspended in 0.4% methylcellulose vehicle. The compositions (given in dosages listed in the left-hand column of Table II below) were administered intraperitoneally either individually or in indicated combinations 30 minutes before challenge with albumin to sensitized mice. Death was recorded 1 hour after the challenge. The results are listed in Table II below.

TABLE II

SYNERGISTIC PROTECTION OF ANAPHYLACTIC DEATH IN SENSITIZED MICE BY COMBINATIONS OF THE INVENTION

| Treatment | Number of Animals[a] | % Survival[a] |
|---|---|---|
| Saline | 38 | 5 |
| A (50 mg/kg) | 16 | 0 |
| B (5 mg/kg) | 11 | 0 |
| C (3 mg/kg) | 24 | 16 |
| A (50 mg/kg) + C (3 mg/kg) | 19 | 84 |
| B (5 mg/kg) + C (3 mg/kg) | 8 | 100 |
| E (0.2 mg/kg) | 18 | 45 |
| A (50 mg/kg) + E (0.2 mg/kg) | 10 | 100 |
| B (5 mg/kg) + E (0.2 mg/kg) | 8 | 100 |
| D (0.1 mg/kg) | 14 | 40 |
| A (50 mg/kg) + D (0.1 mg/kg) | 6 | 100 |
| B (5 mg/kg) + D (0.1 mg/kg) | 8 | 90 |

[a] Pooled data from five separate experiments.

The above results demonstrate that intraveneous administration of PAF (200 μg/kg) induced 100% lethality in mice. PAF antagonists A and B, given intraperitoneally, provided protection against PAF-induced lethality with $ED_{50}$ values of 20 mg/kg and 1.5 mg/kg, respectively (Table I). Neither chlorpheniramine, clemastine nor ketotifen affected PAF-induced mortality (Table I).

When sensitized animals were challenged intraveneously with ovalbumin, nearly 100% of the animals died within 10-20 minutes. The anti-PAF agents A and B, given intraperitoneally at doses as high as 50 mg/kg and 20 mg/kg, respectively, were totally ineffective in protecting these animals from death (Table II). The antihistamines chloropheniramine, clemastine and ketotifen, given individually, provided significant protection (Table II). This protection varied between 30% and 70% depending on the compound, dose and the batch of animals.

When PAF antagonist A at a ineffective dose, was coadministered with a partially effective dose of either chloropheniramine, clemastine or ketotifen, the sensitized animals were completely protected from antigen-induced death (Table II). Similar synergistic protection was observed when an ineffective dose of PAF antagonist B was combined with partially effective doses of the individual antihistamines (Table II).

The compositions of the present invention may be administered employing the PAF antagonist together with or separate from the antihistamine. Preferably, a single dosage form is employed. The dosage of the antihistamine is preferably from 35% to 100% of the antihistamine's normal or effective dose ($ED_{50}$), more preferably from 50% to 100% of the antihistamine's effective dose. The PAF antagonist is preferably administered in amount of from 35% to 200% of its PAF antagonist effective dose ($ED_{50}$) and more preferably from 50% to 150% of the effective dose. For example, compound B above has an $ED_{50}$ (p.o.) in guinea pigs (PAF-induced bronchospasm) of 0.1 mg/kg. Similarly compound A above and alprazolam have $ED_{50}$'s (i.v.) in guinea pigs of 0.4 mg/kg and 3 mg/kg, respectively.

The compositions may be administered orally, i.v., etc., and in general by any of the modes by which the individual components may be administered. Of course, the dose will be regulated according to the potency of individual compounds employed, the mode of administration, and the judgment of the attending clinician depending on factors such as the degree and the severity of the disease state and age and general condition of the patient being treated.

The compositions of the invention can be administered in unit dosage forms such as tablets, capsules, pills, powders, granules, sterile parenteral solutions or suspensions, suppositories or transdermal compositions.

Such dosage forms are prepared according to standard techniques well known in the art.

For preparing pharmaceutical compositions from the compounds described by this invention, inert, pharmaceutically acceptable carriers can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 to 70 percent of the active ingredient. Suitable solid carriers are magnesium carbonate, magnesium stearate, talc, sugar, lactose, pectin, dextrin, starch, gelatin, tragacanth, methylcellulose, sodium carboxymethyl-cellulose, a low melting wax and cocoa butter. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution in polyethylene glycol and/or propylene glycol, which may contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins, methylcellulose, sodium carboxymethylcellulose and other well-known suspending agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners and solubilizing agents. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

## Claims

1. The synergestic use of a PAF antagonist of formula Ia, Ib or II below in combination with an antihistamine for the manufacture of a medicament for treating allergy, wherein the PAF-antagonist has the structural

formula

(1) a PAF-antagonist

(a) of the formula Ia or Ib

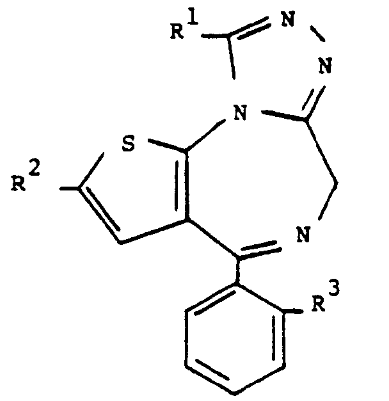

Ia

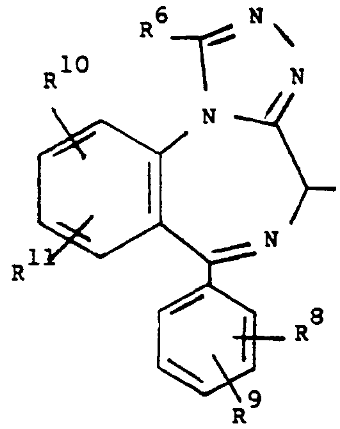

Ib

or a pharmaceutically acceptable salt or solvate of a compound Ia or Ib, wherein:

$R^1$ is H, lower alkyl of 1 to 4 carbon atoms, cyclopropyl, methoxy, chloro or bromo, $R^2$ represents

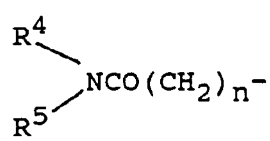

or halo,

$R^3$ represents H, chloro or bromo,

$R^4$ and $R^5$ may be the same or different and each is independently selected from H, alkyl of 1 to 4 carbon atoms or hydroxyalkyl having from 1 to 4 carbon atoms, or $R^4$ and $R^5$ together with the nitrogen atom attached thereto represent a 5, 6 or 7 membered, saturated ring, which ring optionally contains another heteroatom or hetero group selected from -O-, -S-, -NH- or $-N(CH_3)-$,

n represents 0, 1, 2, 3, 4, 5, 6, 7 or 8,

$R^6$ represents H, lower alkyl of 1 to 3 carbon atoms, phenyl, benzyl or COOR′,

$R^7$ represents H or alkyl of 1 to 3 carbon atoms,

$R^8$, $R^9$, $R^{10}$ and $R^{11}$ may be the same or different and each independently represents H; alkyl of 1 to 3 carbon atoms, halo, nitro, cyano, trifluoromethyl, alkoxy, alkylthio, alkylsulfinyl, alkylsulfonyl, alkanoylamino or dialkylamino, wherein each alkyl portion of alkoxy, alkylthio, alkylsulfinyl, alkylsulfonyl, alkanoylamine or dialkylamino contains 1 to 3 carbon atoms,

R′ represents alkyl of 1 to 4 carbon atoms; or

(b) of formula II

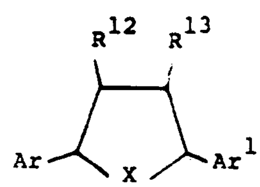

II

wherein:

X is 0, S, SO, or $SO_2$,

$R^{12}$ and $R^{13}$ may be the same or different and each independently represents H, lower alkyl of 1 to 6 carbon atoms, halo, halo-lower alkyl of 1 to 6 carbon atoms, $CONR^{14}R^{15}$, lower alkenyl of 1 to 6 carbon atoms, lower alkynyl of 1 to 6 carbon atoms, $COR^{14}$, $COOR^{14}$, $CH_2OR^{14}$, $CH_2NR^{14}R^{15}$, $CH_2SR^{14}$, =O or $OR^{15}$,

Ar and $Ar^1$ may be the same or different and each independently represents pyrryl, furyl, pyridyl, thienyl, cyclohexyl, naphthyl or a group of the formula

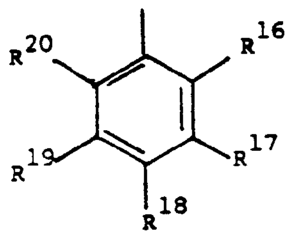

$R^{14}$ and $R^{15}$ may be the same or different and each is independently H or lower alkyl of 1 to 6 carbon atoms;

$R^{16}$, $R^{17}$, $R^{18}$, $R^{19}$ and $R^{20}$ may be the same or different and each isindependently H, $-OR^{14}$, $-SR^{14}$, $-SOR^{14}$, $-SO_2R^{14}$, $-OCF_3$, $-SCF_3$, $-NR^{14}R^{15}$, $-OCH_2CO_2R^{14}$, $-SO_2NR^{14}R^{15}$, $-CO_2R^{14}$, $-N(R^{14})SO_2R^{15}$, $COR^{14}$, $NO_2$ or CN, or $R^{16}$ and $R^{17}$, $R^{17}$ and $R^{18}$, $R^{18}$ and $R^{19}$ or $R^{19}$ and $R^{20}$ together represent a $-OCH_2O-$, $-OCH_2CH_2O-$ or $-OCH_2CH_2NR^{14}-$ bridge.

2. A use according to claim 1, characterized in that the antihistamine is selected from chlorpheniramine, brompheniramine, clemastine, ketotifen, azatadine, loratadine, terfenadine, cetirizine, astemizole, tazifylline, levocabastine, diphenhydramine, temelastine, etolatifen, acrivastine, azelastine, ebastine, mequitazine, 3-amino-9,13b-dihydro-1H-dibenz[c,f]imidazo [1,5-a]azepine, 1-(2-ethoxyethyl)-2-(4-methyl-1-homopiperazinyl)benzimidazole, 2-(dimethylamino)ethyl-2,3-dihydro-4-methylpyrido-[3,2-f]-1,4-oxazepine-5(4H)-thione, or a pharmaceutical acceptable salt of such a compound.

3. The use according to claim 2, characterized in that the PAF antagonist is a compound of formula Ia wherein $R^1$ is $CH_3$; $R^3$ is chloro or bromo; and $R^2$ is bromo or $R^4R^5NCO(CH_2)_n-$.

4. The use according to claim 1 or 2, characterized in that the compound of formula Ia or Ib is selected from

8-chloro-1-methyl-6-phenyl-4H-s-triazolo(4,3-a][1,4]benzodiazepine,

8-chloro-1-methyl-6-(o-fluorophenyl)-4H-s-triazolo[4,3-a][1,4]benzodiazepine,

1-methyl-6-phenyl-4H-s-triazolo[4,3-a][1,4]benzodiazepine,

8-chloro-1-propyl-6-phenyl-4H-s-triazolo(4,3-a]-[1,4]benzodiazepine,

8-chloro-1-isopropyl-6-phenyl-4H-s-triazolo(4,3-a][1,4]benzodiazepine,

8-chloro-1-ethyl-6-phenyl-4H-s-triazolo(4,3-a][1,4]benzodiazepine,

1-methyl-6-phenyl-8-(trifluoromethyl)-4H-s-triazolo[4,3-a][1,4]benzodiazepine,

8-chloro-1-methyl-6-(o-chlorophenyl)-4H-s-triazolo[4,3-a][1,4]benzodiazepine,

4-(2-chlorophenyl)-9-methyl-6H-thieno[3,2-f][1,2,4]triazolo[4,3-a][1,4]diazepine-2-propionic acid morpholide,

4-(2-chlorophenyl)-9-methyl-6H-thieno[3,2-f][1,2,4]triazolo[4,3-a][1,4]diazepine-2-propionamide,

4-(2-chlorophenyl)-9-cyclopropyl-6H-thieno[3,2-f][1,2,4]triazolo[4,3-a][1,4]diazepine-2-propionic acid morpholide, or

4-(2-chlorophenyl)-9-methyl-6H-thienor[3,2-f]-[1,2,4]triazolo[4,3-a][1,4]diazepine-2-propionic acid diethyl amide.

5. The use according to claim 1 or 2, characterized in that the compound of formula I is 4-(2-chlorophenyl)-9-methyl-6H-thieno[3,2-f][1,2,4]triazolo[4,3-a][1,4]diazepine-2-propionic acid morpholide.

6. The use according to claim 1 or 2, characterized in that the PAF antagonist in a compound of formula IIa, IIb or IIc

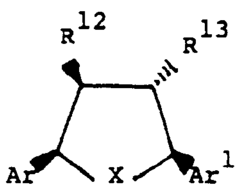
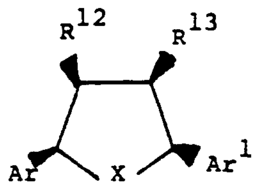
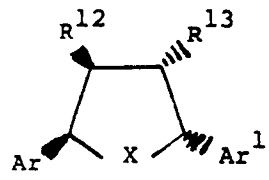

IIa IIb IIc

wherein X, $R^{12}$, $R^{13}$, Ar and $Ar^1$ are as defined in claim 2.

7. The use according to claim 1 or 2, characterized in that the PAF antagonist is selected from
2,5-bis(3,4-dimethoxyphenyl)tetrahydrofuran
2,5-bis(3,4,5-trimethoxyphenyl)tetrahydrofuran,
2,5-bis(3,4,5-trimethoxyphenyl)tetrahydrothiophene, or
2-(3-methoxy-5-methylsulfonyl-4-propoxyphenyl)-5-(3,4,5-trimethoxyphenyl)tetrahydrofuran.

8. The use according to claim 7, characterized in that the compound of formula II is in its trans form.

9. A synergistic pharmaceutical composition suitable for treating allergic reactions comprising an anti-allergic effective amount of a combination of a PAF-antagonist of formula Ia, Ib or II as defined in claim 1 in combination with an antihistamine.

10. A process for the preparation of a pharmaceutical composition as claimed in claim 9 which comprises admixing a PAF-antagonist as defined in any one of claims 1, 2 or 3-8, an antihistamine an a pharmaceutically acceptable carrier.

**Patentansprüche**

1. Synergistische Verwendung eines PAF Antagonisten der nachstehenden Formeln Ia, Ib oder II in Kombination mit einem Antihistamin zur Herstellung eines Medikaments zur Behandlung von Allergien, wobei der PAF-Antagonist die Strukturformel hat
(1) ein PAF-Antagonist
(a) der Formel Ia oder Ib

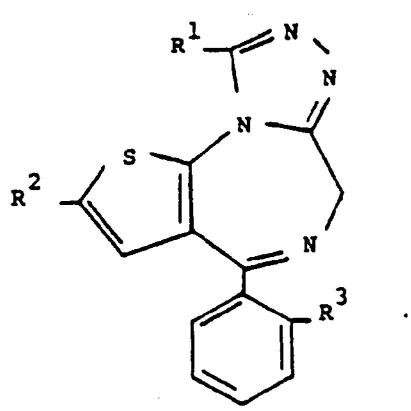
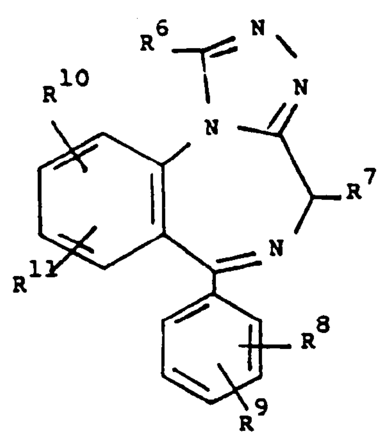

Ia Ib

oder eines pharmazeutisch annehmbaren Salzes oder eines Solvates der Verbindungen Ia oder Ib,

worin:

$R^1$ H, Niederalkyl mit 1 bis 4 Kohlenstoffatomen, Cyclopropyl, Methoxy, Chlor, oder Brom ist,

$R^2$

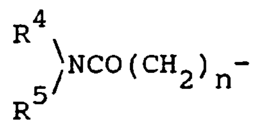

oder Halogen bedeutet,

$R^3$ H, Chlor, oder Brom bedeutet,

$R^4$ und $R^5$ gleich oder verschieden sein können und unabhängig voneinander aus H, Alkyl mit 1 bis 4 Kohlenstoffatomen, oder Hydroxyalkyl mit 1 bis 4 Kohlenstoffatomen ausgewählt sind, oder $R^4$ und $R^5$ zusammen mit dem Stickstoffatom, an das sie gebunden sind, einen 5, 6 oder 7-gliedrigen gesättigten Ring darstellen, wobei der Ring wahlweise ein weiteres Heteroatom oder eine weitere Heterogruppe enthält, die aus -O-, -S-, -NH- oder $-N(CH_3)-$, gewählt wird,

n 0, 1, 2, 3, 4, 5, 6, 7 oder 8 bedeutet,

$R^6$ H, Niederalkyl mit 1 bis 3 Kohlenstoffatomen, Phenyl, Benzyl oder COOR′ bedeutet,

$R^7$ H oder Alkyl mit 1 bis 3 Kohlenstoffatomen bedeutet,

$R^8$, $R^9$, $R^{10}$ und $R^{11}$ gleich oder verschieden sein können und jeweils unabhängig voneinander H, Alkyl mit 1 bis 3 Kohlenstoffatomen, Halogen, Nitro, Cyano, Trifluoromethyl, Alkoxy, Alkylthio, Alkylsulfinyl, Alkylsulfonyl, Alkanoylamino oder Dialkylamino bedeuten, worin jeder Alkylrest von Alkoxy, Alkylthio, Alkylsulfinyl, Alkylsulfonyl, Alkanoylamino oder Dialkylamino, 1 bis 3 Kohlenstoffatome enthält,

R′ Alkyl mit 1 bis 4 Kohlenstoffatomen bedeutet; oder

(b) der Formel II

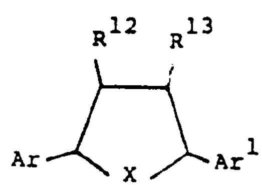

II

worin:

X O, S, SO oder $SO_2$ ist,

$R^{12}$ und $R^{13}$ gleich oder verschieden sein können und unabhängig voneinander H, Niederalkyl mit 1 bis 6 Kohlenstoffatomen, Halogen, Halogen-Niederalkyl mit 1 bis 6 Kolenstoffatomen, $CONR^{14}R^{15}$, Niederalkenyl mit 1 bis 6 Kohlenstoffatomen, Niederalkinyl mit 1 bis 6 Kohlenstoffatomen, $COR^{14}$, $COOR^{14}$, $CH_2OR^{14}$, $CH_2NR^{14}R^{15}$, $CH_2SR^{14}$, =O oder $OR^{15}$ darstellen,

Ar und $Ar^1$ gleich oder verschieden sein können und unabhängig voneinander Pyrryl, Furyl, Pyridyl, Thienyl, Cyclohexyl, Naphthyl oder eine Gruppierung der Formel

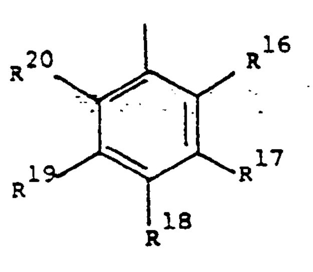

darstellen,

$R^{14}$ und $R^{15}$ gleich oder verschieden sein können und unabhängig voneinander H, oder Niederalkyl mit 1 bis 6 Kohlenstoffatomen darstellen;

$R^{16}$, $R^{17}$, $R^{18}$, $R^{19}$ und $R^{20}$ gleich oder verschieden sein können und unabhängig voneinander H, $-OR^{14}$, $-SR^{14}$, $-SOR^{14}$, $-SO_2R^{14}$, $-OCF_3$, $-SCF_3$, $-NR^{14}R^{15}$, $-OCH_2CO_2R^{14}$, $-SO_2NR^{14}R^{15}$, $-CO_2R^{14}$, $-N(R^{14})SO_2R^{15}$, $COR^{14}$ , $NO_2$ oder CN darstellen, oder $R^{16}$ und $R^{17}$, $R^{17}$ und $R^{18}$, $R^{18}$ und $R^{19}$ oder $R^{19}$ und $R^{20}$ zusammen eine $-OCH_2O-$, $-OCH_2CH_2O-$ oder $-OCH_2CH_2NR^{14}$ Brücke darstellen.

2. Verwendung gemäß Anspruch 1, dadurch gekennzeichnet, daß das Antihistamin aus Chlorpheniramin, Brompheniramin, Clemastin, Ketotifen, Azatadin, Loratadin, Terfenadin, Cetirizin, Astemizol, Tazifyllin, Levocabastin, Diphenhydramin, Temelastin, Etolatifen, Acrivastin, Azelastin, Ebastin, Mequitazin, 3-Amino-9, 13b-dihydro-1H-dibenz[c,f]imidazo[1,5-a]azepin, 1-(2-Ethoxyethyl)-2-(4-methyl-1-homopiperazinyl)benzimidazol, 2-(Dimethylamino)ethyl-2,3-dihydro-4methylpyrido-[3,2f]-1,4-oxazepin-5(4H)-thion, oder einem pharmazeutisch annehmbaren Salz einer solchen Verbindung ausgewählt ist.

3. Verwendung gemäß Anspruch 2, dadurch gekennzeichnet, daß der PAF Antagonist eine Verbindung der Formel Ia ist, worin $R^1$ $CH_3$ ist; $R^3$ Chlor oder Brom ist; und $R^2$ Brom oder $R^4R^5NCO(CH_2)_n-$ ist.

4. Verwendung gemäß Anspruch 1 oder 2, dadurch gekennzeichnet, daß die Verbindung der Formel Ia oder Ib aus folgenden ausgewählt wird
   8-Chlor-1-methyl-6-phenyl-4H-s-triazolo[4,3-a][1,4]benzodiazepin,
   8-Chlor-1-methyl-6-(o-fluorphenyl)-4H-s-triazolo[4,3-a][1,4]benzodiazepin,
   1-Methyl-6-phenyl-4H-s-triazolo[4,3-a][1,4]benzodiazepin,
   8-Chlor-1-propyl-6-phenyl-4H-s-triazolo[4,3-a][1,4]benzodiazepin,
   8-Chlor-1-isopropyt-6-phenyl-4H-s-triazolo[4,3-a][1,4]benzodiazepin,
   8-Chlor-1-ethyl-6-phenyl-4H-s-triazolo[4,3-a][1,4]benzodiazepin,
   1-Methyl-6-phenyl-8-(trifluormethyl)-4H-s-triazolo[4,3-a][1,4]benzodiazepin,
   8-Chlor-1-methyl-6-(o-chtorphenyl)-4H-s-triazolo[4,3-a][1,4]benzodiazepin,
   4-(2-Chlorphenyl)-9-methyl-6H-thieno[3,2-f]-[1,2,4]triazolo[4,3-a][1,4]diazepin-2-propionsäuremorpholid,
   4-(2-Chlorphenyl)-9-methyl-6H-thieno[3,2-f]-[1,2,4]triazolo[4,3-a][1,4]diazepin-2-propionamid,
   4-(2-Chlorphenyl)-9-cyclopropyl-6H-thieno[3,2-f]-[1,2,4]triazolo[4,3-a][1,4]diazepin-2-propionsäuremorpholid, oder
   4-(2-Chlorphenyl)-9-methyl-6H-thieno[3,2-f]-[1,2,4]triazolo[4,3-a][1,4]diazepin-2-propionsäurediethylamid.

5. Verwendung gemäß Anspruch 1 oder 2, dadurch gekennzeichnet, daß die Verbindung der Formel I 4-(2-Chlorphenyl)-9-methyl-6H-thieno[3,2-f][1,2,4]triazolo[4,3-a][1,4]-diazepin-2-propionsäuremorpholid ist.

6. Verwendung gemäß Anspruch 1 oder 2, dadurch gekennzeichnet, daß der PAF Antagonist eine Verbindung der Formel IIa, IIb oder IIc ist,

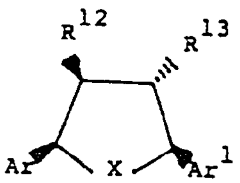
IIa

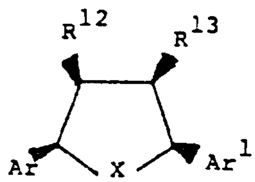
IIb

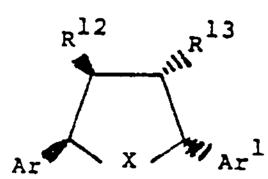
IIc

worin X, $R^{12}$, $R^{13}$, Ar und $Ar^1$ die in Anspruch 2 definierte Bedeutung haben.

7. Verwendung gemäß Anspruch 1 oder 2, dadurch gekennzeichnet, daß der PAF Antagonist aus
   2,5-Bis(3,4-dimethoxyphenyl)tetrahydrofuran,
   2,5-Bis(3,4,5-trimethoxyphenyl)tetrahydrofuran,
   2,5-Bis(3,4,5-trimethoxyphenyl)tetrahydrothiophen, oder
   2-(3-Methoxy-5-methylsulfonyl-4-propoxyphenyl)-5-(3,4,5-trimethoxyphenyl)tetrahydrofuran ausgewählt wird.

8. Verwendung gemäß Anspruch 7, dadurch gekennzeichnet, daß die Verbindung der Formel II in ihrer trans-Form vorliegt.

9. Synergistische pharmazeutische Zusammensetzung geeignet zur Behandlung allergischer Reaktionen, umfassend eine antiallergisch wirksame Menge einer Kombination aus einem PAF-Antagonisten der Formel Ia, Ib oder II, wie in Anspruch 1 definiert, und einem Antihistamin.

10. Verfahren zur Herstellung einer pharmazeutischen Zusammensetzung wie in Anspruch 9 gefordert, umfassend das Zusammenmischen eines PAF-Antagonisten, wie in einem der Ansprüche 1, 2 oder 3 bis 8 definiert, einem Antihistamin und einem pharmazeutisch annehmbaren Träger.

## Revendications

1. Utilisation synergique d'un antagoniste du PAF de formule Ia, Ib ou II ci-après en combinaison avec un anti-histaminique, pour la fabrication d'un médicament destiné au traitement de l'allergie, où l'antagoniste du PAF a la formule développée suivante :
   (1) un antagoniste du PAF
   (a) de formule Ia ou Ib

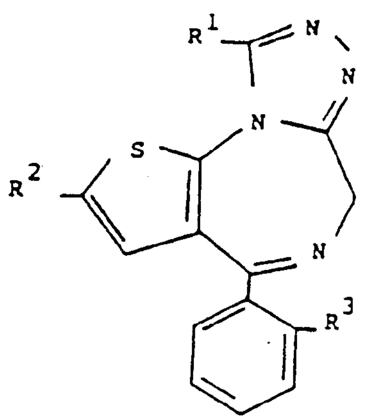

Ia

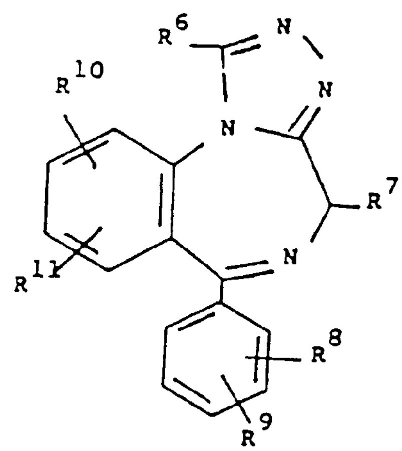

Ib

ou encore un sel ou un produit de solvatation acceptable d'un point de vue pharmaceutique, d'un composé de formule Ia ou Ib, où

$R^1$ est H ou un radical alkyle inférieur ayant 1 à 4 atomes de carbone, cyclopropyle, méthoxy, chloro ou bromo,

$R^2$ représente

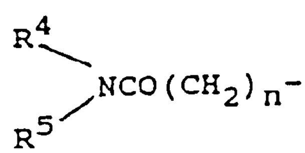

ou halogéno,

$R^3$ représente H, chloro ou bromo,

$R^4$ et $R^5$, qui peuvent être identiques ou différents, sont chacun indépendamment de l'autre choisis parmi H et les radicaux alkyle ayant 1 à 4 atomes de carbone ou hydroxyalkyle ayant 1 à 4 atomes de carbone, ou encore $R^4$ et $R^5$, avec l'atome d'azote auquel ils sont liés, représentent un noyau saturé à 5, 6 ou 7 chaînons, lequel noyau contient éventuellement un autre hétéro-atome ou un autre hétéro-groupe choisi parmi -O-, -S-, -NH- ou $-N(CH_3)-$,

n représente 0, 1, 2, 3, 4, 5, 6, 7 ou 8,

$R^6$ représente H ou un radical alkyle inférieur ayant 1 à 3 atomes de carbone, phényle, benzyle ou COOR′,

$R^7$ est H ou un radical alkyle ayant 1 à 3 atomes de carbone,

$R^8$, $R^9$, $R^{10}$ et $R^{11}$ peuvent être identiques ou différents et représentent chacun indépendamment de l'autre un hydrogène ou un radical alkyle ayant 1 à 3 atomes de carbone, halogéno, nitro, cyano, trifluorométhyle, alcoxy, alkylthio, alkylsulfinyle, alkylsulfonyle, alcanoylamino ou dialkylamino, chaque portion alkyle des groupements alcoxy, alkylthio, alkylsulfinyle, alkylsulfonyle, alcanoylamine ou dialkylamino contenant 1 à 3 atomes de carbone,

R′ est un radical alkyle ayant 1 à 4 atomes de carbone ; ou

(b) de formule II

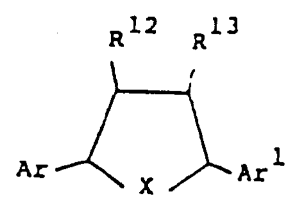

II

où

X est O, S, SO ou $SO_2$,

$R^{12}$ et $R^{13}$ peuvent être identiques ou différents et sont chacun indépendamment de l'autre choisis parmi H et les radicaux alkyles inférieurs ayant 1 à 6 atomes de carbone, halogéno, halogéno-(alkyl inférieur) ayant 1 à 6 atomes de carbone, $CONR^{14}R^{15}$, alcényle inférieur ayant 1 à 6 atomes de carbone, alcynyle inférieur ayant 1 à 6 atomes de carbone, $COR^{14}$, $COOR^{14}$, $CH_2OR^{14}$, $CH_2NR^{14}R^{15}$, $CH_2SR^{14}$, =O ou $OR^{15}$,

Ar et $Ar^1$ peuvent être identiques ou différents et sont chacun indépendamment de l'autre choisis parmi les radicaux pyrryle, furyle, pyridyle, thiényle, cyclohexyle, naphtyle ou un groupe de formule

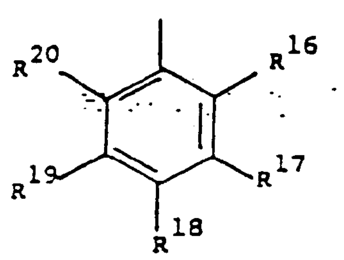

$R^{14}$ et $R^{15}$ peuvent être identiques ou différents et sont chacun indépendamment de l'autre choisis parmi H ou les radicaux alkyle inférieur ayant 1 à 6 atomes de carbone ;

$R^{16}$, $R^{17}$, $R^{18}$, $R^{19}$ et $R^{20}$ peuvent être identiques ou différents et sont chacun indépendamment des autres choisis parmi H, $-OR^{14}$, $-SR^{14}$, $-SOR^{14}$, $-SO_2R^{14}$, $-OCF_3$, $-SCF_3$, $-NR^{14}R^{15}$, $-OCH_2CO_2R^{14}$, $-SO_2NR^{14}R^{15}$, $-CO_2R^{14}$, $-N(R^{14})SO_2R^{15}$, $COR^{14}$, $NO_2$ ou CN, ou $R^{16}$ et $R^{17}$, $R^{17}$ et $R^{18}$, $R^{18}$ et $R^{19}$ ou $R^{19}$ et $R^{20}$ forment ensemble un pont $-OCH_2O-$, $-OCH_2CH_2O-$ ou $-OCH_2CH_2NR^{14}$.

2. Utilisation selon la revendication 1, caractérisée en que l'anti-histaminique est choisi parmi les composés suivants : la chlorphéniramine, la bromphéniramine, la clémastine, le kétotifène, l'azatadine, la loratadine, la terfénadine, la cétirizine, l'astémizole, la tazifylline, la lévocabastine, la diphènhydramine, la témélastine, l'étolotifène, l'acrivastine, l'azélastine, l'ébastine, la méquitazine, 3-amino-9,13b-dihydro-1H-dibenzo[c,f]imidazo[1,5-a]azépine, 1-(2-éthoxyéthyl)-2-(4-méthyl-1-homopipérazinyl)-benzimidazole, 2-(diméthyl-amino)éthyl-2,3-dihydro-4-méthylpyrido[3,2-f]1,4- oxazépine-5(4H)-thione, ou un sel pharmaceutiquement acceptable de ces composés.

3. Utilisation selon la revendication 2, caractérisée en ce que l'antagoniste du PAF est un composé de formule Ia dans laquelle $R^1$ est $CH_3$ ; $R^3$ est le radical chloro ou bromo ; et $R^2$ est le radical bromo ou un radical $R^4R^5NCO(CH_2)_n$-.

4. Utilisation selon la revendication 1 ou 2, carctérisée en ce que le composé de formule Ia ou Ib est choisi parmi les composés suivants :
8-chloro-1-méthyl-7-phényl-4H-s-triazolo[4,3-a][1,4]benzodiazépine,
8-chloro-1-méthyl-6-(o-fluorophényl)-4H-s-triazolo[4,3-a][1,4]benzodiazépine,
1-méthyl-6-phényl-4H-s-triazolo[4,3-a][1,4]benzodiazépine,
8-chloro-1-propyl-6-phényl-4H-s-triazolo[4,3-a][1,4]benzodiazépine,
8-chloro-1-isopropyl-6-phényl-4H-s-triazolo[4,3-a][1,4]benzodiazépine,
8-chloro-1-éthyl-6-phényl-4H-s-triazolo[4,3-a][1,4]benzodiazépine,
1-méthyl-6-phényl-8-(trifluorométhyl)-4H-s-triazolo[4,3-a][1,4]benzodiazépine,
8-chloro-1-méthyl-6-(o-chlorophényl)-4H-s-triazolo[4,3-a][1,4]benzodiazépine,
morpholide de l'acide 4-(2-chlorophényl)-9-méthyl-6H-thiéno[3,2-f][1,2,4]triazolo[4,3-a][1,4]diazépine-2-propionique,
4-(2-chlorophényl)-9-méthyl-6H-thiéno[3,2-f][1,2,4]triazolo[4,3-a][1,4]diazépine-2-propionamide,
morpholide de l'acide 4-(2-Chlorophényl)-9-cyclopropyl-6H-thiéno-[3,2-f][1,2,4]triazolo[4,3-a][1,4]diazépine-2-propionique, ou
diéthylamide de l'acide 4-(2-chlorophényl)9-méthyl-6H-thiéno[3,2-f][1,2,4]triazolo[4,3-a][1,4]diazépine-2-propionique.

5. Utilisation selon la revendication 1 ou 2, caractérisée en ce que le composé de formule I est le morpholide de l'acide 4-(2-chlorophényl)-9-méthyl-6H-thiéno[3,2-f][1,2,4]triazolo[4,3-a][1,4]diazépine-2-propionique.

6. Utilisation selon la revendication 1 ou 2, caractérisée en ce que l'antagoniste du PAF est un composé de formule IIa, IIb ou IIc

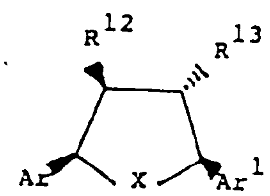

IIa

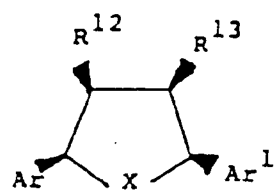

IIb

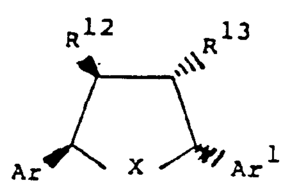

IIc

où X, $R^{12}$, $R^{13}$, Ar et $Ar^1$ sont tels que définis dans la revendication 2.

7. Utilisation selon la revendication 1 ou 2, caractérisée en ce que l'antagoniste du PAF est choisi parmi les composés suivants :
2,5-bis(3,4-diméthoxyphényl)tétrahydrofuranne,
2,5-bis(3,4,5-triméthoxyphényl)tétrahydrofuranne,
2,5-bis(3,4,5-triméthoxyphényl)tétrahydrothiophène, ou 2-(3-méthoxy-5-méthylsulfonyl-4-propoxyphényl)-5-(3,4,5-triméthoxyphényl)tétrahydrofuranne.

8. Utilisation selon la revendication 7, caractérisée en ce que le composé de formule II se présente sous sa forme trans.

9. Composition pharmaceutique synergique adaptée au traitement des réactions allergiques, comprenant une quantité à effet anti-allergique d'une combinaison d'un antagoniste du PAF de formule Ia, Ib ou II selon la revendication 1, et d'un anti-histaminique.

10. Procédé de préparation d'une composition pharmaceutique selon la revendication 9, qui consiste à mélanger un antagoniste du PAF selon l'une quelconque des revndications 1, 2 ou 3 à 8, un anti-histaminique et un excipient pharmaceutiquement acceptable.